Europäisches Patentamt

**European Patent Office**

**Office européen des brevets**

(11) Veröffentlichungsnummer: **0 155 676**

**A2**

(12) **EUROPÄISCHE PATENTANMELDUNG**

(21) Anmeldenummer: **85103193.0**

(22) Anmeldetag: **19.03.85**

(51) Int. Cl.⁴: **C 07 K 15/06**
**G 01 N 33/68, G 01 N 33/57-**
**7**

(30) Priorität: **19.03.84 DE 3410049**
**07.09.84 DE 3433021**

(43) Veröffentlichungstag der Anmeldung:
**25.09.85 Patentblatt 85/39**

(84) Benannte Vertragsstaaten:
**AT BE CH DE FR GB IT LI LU NL SE**

(71) Anmelder: **Max-Planck-Gesellschaft zur Förderung der Wissenschaften e.V.**
**Bunsenstrasse 10**
**D-3400 Göttingen(DE)**

(72) Erfinder: **Timpl, Rupert, Dr.**
**Julius-Haerlin-Strasse 3**
**D-8035 Gauting(DE)**

(72) Erfinder: **Brocks, Dietrich, Dr.**
**Buchenstrasse 1**
**D-6257 Hünfelden 1(DE)**

(74) Vertreter: **Huber, Bernhard, Dipl.-Chem. et al,**
**Patentanwälte H. Weickmann, Dr. K. Fincke F.A.**
**Weickmann, B. Huber Dr. H. Liska, Dr. J. Prechtel**
**Möhlstrasse 22 Postfach 860 820**
**D-8000 München 86(DE)**

(54) **Verfahren zur Gewinnung der globulären Domäne von Basalmembrankollagen und immunologische Bestimmung von Basalmembranmaterial und Autoantikörpern.**

(57) Die humane globuläre Basalmembrankollagen-Domäne NCI läßt sich gewinnen, indem man menschliches oder tierisches Gewebe einer ersten limitierenden Behandlung mit bakterieller Kollagenase unterwirft, die erhaltenen Abbauprodukte durch Chromatographie an schwach basischem Anionenaustauscher von nichtkollagenen Proteinen abtrennt, die kollagenen Abbauprodukte dann einem zweiten Kollagenaseabbau bei erhöter Temperatur unterwirft und die globuläre Domäne NCI durch Molekularsiebfraktionierung reinigt und weist ein Molekulargewicht von etwa 170000 Dalton, bestimmt durch Ultrazentrifugierung, hexamere Struktur mit monomeren Untereinheiten von Molekulargewicht etwa 28000 Dalton und einen Kohlenhydratgehalt von etwa 2 % auf. Sie eignet sich zur Bestimmung von Basalmembrankollagen in Körperflüssigkeiten bei Verwendung ihrer Antikörper sowie zum Nachweis von hiergegen gerichteten Antikörpern in Körperflüssigkeiten.

B e s c h r e i b u n g

Die Erfindung betrifft die humane globuläre Domäne NC1
von Basalmembrankollagen (Typ IV), ein einfaches
Verfahren zur Isolierung der Domäne NC1 aus verschiedenen menschlichen und tierischen Geweben und die Verwendung des erwähnten humanen Proteinfragments zur
immunologischen Bestimmung von Basalmembrankomponenten
in Körperflüssigkeiten und von Autoimmunreaktionen
gegen Basalmembranen.

Bei einer großen Zahl von z. T. sehr häufigen Erkrankungen (Diabetes, Vasculitis, Mephropathien, Neurofibrom,
Leberfibrose, epitheliale Tumoren) kommt es zu einer
Veränderung in Basalmembranen (Verdickung, Auflösung)
vor allem in den Gefäßwänden. Diese Veränderungen stellen oft letale Komplikationen dar. Der Nachweis solcher
Veränderungen kann mittels Elektronenmikroskopie oder
Immunfluoreszenz erfolgen, erfordert jedoch Biopsiematerial und erlaubt nur qualitative Aussagen. Es
konnte kürzlich an entsprechenden Tiermodellen (z. B.
Streptozotozin-Diabetes der Ratte) gezeigt werden, daß
sich solche Basalmembranveränderungen relativ früh in
einer Erhöhung von Basalmembrankomponenten in der
Zirkulation oder in anderen Körperflüssigkeiten manifestieren.

Eine weitere Form von pathologischen Basalmembranveränderungen beruht auf Autoimmunreaktionen gegen körpereigene Basalmembranproteine, die über Nachfolgereaktionen (Entzündung, proteolytischer Abbau) zu einer
Beeinträchtigung der Funktion von Basalmembranen in verschiedenen Organen (z. B. Niere, Lunge) führen kann.
Solche Erkrankungen sind beim Menschen verschiedene
chronische Nierenerkrankungen, multiple Sklerose,
Diabetes und einige seltene Erkrankungen wie Goodpasture
Syndrom und bullöses Pemphigoid. Es ist zu vermuten,
daß viele dieser Autoimmunreaktionen infolge des
Mangels an geeigneten empfindlichen Testverfahren
bisher nicht entdeckt wurden. Zahlreiche Tierversuche
zeigten jedoch, daß einige der Basalmembrankomponenten
autoantigene Eigenschaften besitzen, oder daß Antikörper
gegen diese Proteine pathologische Veränderungen
bewirken.

Basalmembrankollagen (Typ IV) ist eine in allen Basalmembranen vorkommende Komponente, und neben anderen
Funktionen vor allem für die mechanische Stabilität
dieser Strukturen verantwortlich. Eine erhöhte Synthese
oder Zerstörung von Basalmembranen sollte daher mit
einem erhöhten Auftreten von Strukturen des Basalmembrankollagens, z. B. in der Zirkulation verbunden sein.
Basalmembrankollagen mit einem Molekulargewicht von ca.
600 000 besteht aus verschiedenen strukturellen
Domänen: einer ca. 330 nm langen Tripelhelix, einer
weiteren kurzen (60 nm) Tripelhelix (7S-Domäne) und
einer globulären Domäne NC1. Der größte Teil des Basalmembrankollagens des Gewebes ist infolge kovalenter
Quervernetzungen unlöslich. Diese Quervernetzungen
werden nach Assoziation der Moleküle zu großen, vermutlich regulären Netzwerken ausgebildet. Die Assoziation
erfolgt dabei über die 7S-Domäne und die globuläre
Domäne NC1, wobei jeweils vier bzw. zwei Moleküle miteinander verknüpft werden. Infolge dieser Anordnung ist
die globuläre Domäne NC1 des Gewebekollagens jeweils
das Assoziat aus zwei verschiedenen Molekülen.

Der Erfindung liegt nun die Aufgabe zugrunde, die humane globuläre Basalmembrankollagen-Domäne NC1 zur Verfügung zu stellen, ein allgemein anwendbares Verfahren zur Isolierung der globulären Domäne NC1 von Basalmembrankollagen zu entwickeln und dieses Material humanen Ursprungs nach entsprechender Markierung zum empfindlichen und quantitativen Nachweis von Basalmembrankollagen in Körperflüssigkeiten einzusetzen. Gleichzeitig soll es die Erfindung ermöglichen, biologische Flüssigkeiten oder Gewebe auf das Vorliegen von Autoantikörpern oder autoreaktiven Immunzellen zu untersuchen.

Gelöst wird diese Aufgabe erfindungsgemäß durch humane globuläre Basalmembrankollagen-Domäne NC1, welche ein Molekulargewicht von etwa 170000 Dalton, bestimmt durch Gleichgewichts-Ultrazentrifugation und Lichtstreuung, hexamere Struktur mit monomeren Untereinheiten von Molekulargewicht etwa 28000 Dalton und einem Kohlenhydratgehalt von etwa 2 % aufweist.

Ein weiterer Gegenstand der Erfindung ist ein Verfahren zur Gewinnung und Reinigung der globulären Basalmembrankollagen-Domäne NC1, welches dadurch gekennzeichnet ist, daß man menschliches oder tierisches Gewebe einer ersten limitierenden Behandlung mit bakterieller Kollagenase unterwirft, die erhaltenen Abbauprodukte durch Chromatographie an schwach basischem Anionenaustauscher von nichtkollagenen Proteinen abtrennt, die kollagenen Abbauprodukte dann einem zweiten Kollagenaseabbau bei erhöhter Temperatur unterwirft und die globuläre Domäne NC1 durch Molekularsiebfraktionierung reinigt.

Das erfindungsgemäße Verfahren zur Reinigung der globulären Domäne NC1 geht direkt von den Geweben aus und verzichtet auf eine vorherige Reinigung der Basalmembranen, die üblicherweise sehr verlustreich ist. Wesentlich ist eine erste limitierte Behandlung des Gewebes mit bakterieller Kollagenase, die die globuläre Domäne und große Stücke der kollagenen Tripelhelix in intakter Form aus dem Material herauslöst. Diese Komponenten lassen sich dann leicht, z. B. an DEAE Zellulose von begleitenden nicht-kollagenen Proteinen (z. B. Laminin) abtrennen. Der zweite unter verschärften Bedingungen durchgeführte Kollagenaseabbau zerstört dann alle kollagenen, tripelhelikalen Strukturen mit Ausnahme der quervernetzten 7S-Domäne und der globulären Domäne NC1. Beide Komponenten lassen sich voneinander und von begleitenden Abbaupeptiden in einem einzigen Molekularsiebschritt (z. B. an Agarose) separieren und werden dann mit hoher Reinheit erhalten.

Das Verfahren läßt sich auf verschiedene Gewebe anwenden, wobei bevorzugt menschliche Placenta, Niere oder Lunge eingesetzt wird. Die Ausbeute an globulärer Domäne NC1 beträgt dabei im Durchschnitt 20 mg pro kg Feuchtgewebe. Das Verfahren läßt sich auch auf tierische Gewebe anwenden, z. B. auf Rinderaorta bzw. das EHS Sarkom der Maus.

Zweckmäßig geht man beim Verfahren der Erfindung von einem Gewebe aus, welches in salzhaltiger Pufferlösung homogenisiert wurde. Ferner ist es von Vorteil für die spätere Reinigung von NC1, das Gewebe vor der ersten Kollagenasebehandlung mit Guanidinhydrochlorid in Anwesenheit eines Proteaseinhibitors zu extrahieren. Besonders bewährt hat sich dabei eine Extraktion mit einer etwa 0,5 M KCl und danach mit einer etwa 6 M Guanidin enthaltenden Lösung.

Diese Schritte entfernen einen beträchtlichen Teil der Begleitproteine, ohne daß es dabei zu einer Auflösung oder Zerstörung der globulären Domäne NC1 kommt.

Die erste Kollagenasebehandlung wird vorzugsweise bei einer Temperatur von 15 bis 25°C durchgeführt. Die zweite, nicht limitierte Kollagenasebehandlung hingegen wird vorzugsweise bei einer Temperatur von 30 bis 45°C durchgeführt.

Die globuläre Domäne NC1 ist nach der Reinigung in der Ultrazentrifuge einheitlich und besitzt ein Molekulargewicht von 170.000. Die Struktur ist prinzipiell ein Hexamer, wobei die monomeren Untereinheiten ein Molekulargewicht von 28.000 aufweisen. Der größte Teil der Monomeren ist jedoch über Disulfidbrücken oder andere kovalente Quervernetzungen zu dimeren Untereinheiten vereinigt. Das verursacht ein charakteristisches Elektrophoresemuster (in Gegenwart von Na-Dodecylsulfat) von ein bis zwei Monomerenbanden und zwei, üblicherweise stärkeren Dimerenbanden. Diese hexamere Struktur ist unter physiologischen Bedingungen (z. B. neutrale Puffer) stabil auch gegen proteolytischen Abbau. Eine Dissoziation in die Untereinheiten erfolgt bei saurem pH oder in Gegenwart von 8 M Harnstoff. Diese Dissoziation ist weitgehend reversibel. Dialyse gegen neutrale Puffer erlaubt die Rekonstitution zu einer hexameren globulären Struktur.

Die Aminosäurezusammensetzungen der globulären Domäne NC1 aus menschlicher Placenta, Niere oder Lunge sind sehr ähnlich (Tabelle). Der Kohlenhydratgehalt des Materials ist gering (20 %) und beinhaltet sowohl Glukosamin als auch Galaktosamin. Die verschiedenen Untereinheiten der Struktur können sowohl von der
1(IV)- und 2(IV)-Kette des Basalmembrankollagens
stammen, die sich in ihren Anfangssequenzen unterscheiden.

Diese Basalmembranstruktur NC1, die im folgenden als
Antigen bezeichnet wird, ermöglicht die erfindungsgemäße Bestimmung von Basalmembrankollagen im Blut und
anderen Körperflüssigkeiten unter Anwendung von an sich
bekannten immunologischen Nachweismethoden. Diese
Methoden beruhen auf der Konkurrenz einer bekannten
Menge markierten Antigens mit einer unbekannten Menge
des Antigens in der zu untersuchenden Probe um den
gemeinsamen Antikörper. Es können dabei sowohl bekannte
Varianten des Radioimmuntests (RIA) als auch des Enzymimmuntests (EIA) eingesetzt werden. Derartige Methoden
sind dem Fachmann bekannt und werden im einzelnen nicht
nochmals aufgeführt. Alle diese Verfahren beruhen darauf, daß man mit dem hochgereinigten Antigen in geeigneten Versuchstieren Antiseren (Antikörper) herstellt,
und mit diesen oder auch spezifisch isolierten Antikörpern durch entsprechende Inkubation der Reaktionspartner die übliche Antikörper-Antigen-Komplexierungsreaktion ablaufen läßt. Je nach der Menge des in der zu
untersuchenden Probe einer Körperflüssigkeit vorhandenen nichtmarkierten Antigens wird nur ein Teil des
markierten Antigens in diesem Komplex gebunden und kann
entweder durch Isolierung des Komplexes oder im Überstand gemessen werden. Da die Menge des im Komplex
gebundenen markierten Antigens von der Menge des
nicht-markierten Antigens abhängig ist, kann auf diese
Weise der Gehalt an Basalmembrankollagen bzw. der
Domäne NC1 in der Körperflüssigkeit bestimmt werden.

Die Herstellung der Antiseren kann in üblicher Weise durch subkutane Injektion von Versuchstieren, vorzugsweise Kaninchen, erfolgen. Zweckmäßig wird dabei das Antigen zusammen mit kompletten Freund'schen Adjuvans appliziert. Als ausreichend erweist sich dabei die zweimalige Injektion von 0,1 bis 0,2 mg der globulären Domäne NC1 als Antigen. Das gebildete Antiserum wird dann wie üblich gewonnen und kann als solches verwendet werden. Es ist auch möglich, die im Serum vorhandenen Antikörper nach Methoden der Affinitätschromatographie zu reinigen.

Die Markierung des Antigens kann prinzipiell mit bekannten Methoden der Einführung des Radionuklids Jod 125 in Proteine erfolgen. Dabei erweist sich die Markierung der globulären Domäne mit dem Bolton-Hunter-Reagenz (Biochem. J. 133, 529-539, 1973) als besonders vorteilhaft. Die Markierung nach der Chloramin T Methode verläuft weniger erfolgreich und produziert markierte Antigene, die nur noch 20 bis 50 % an den Antikörper binden.

Eine bevorzugte Ausführungsform der erfindungsgemäßen Bestimmung besteht darin, die Trennung des mit dem spezifischen Antiserum gebildeten Antigen-Antikörper-Komplexes von nichtgebundenem Antigen durch Verwendung eines zweiten Antikörpers (Antiserums) durchzuführen. Bevorzugt wird hierbei als zweiter Antikörper ein Antiserum gegen Immunglobulin G der für die Gewinnung des Antiserums verwendeten Tierart eingesetzt. Die Trennung des damit in unlösliche Form überführten Antigen-Antikörper-Komplexes von der Lösung kann nach hierfür üblichen Methoden, wie z. B. Abzentrifugieren, erfolgen. Nach Abtrennung der Antigen-Antikörper-Komplexe wird die Markierung (Radioaktivität, Enzymaktivität), die im Komplex gebunden ist, gemessen. Anhand einer Eichkurve, die mittels Proben von bekanntem Antigengehalt erstellt wurde, läßt sich dann die in der zu untersuchenden Probe enthaltene Menge an Antigen feststellen.

Das erfindungsgemäße immunologische Bestimmungsverfahren erlaubt die Messungen von Konzentrationen bis in den Bereich von 1 ng/ml. Damit ist es möglich, dieses Verfahren zur Feststellung von Basalmembrankollagen in Körperflüssigkeiten einzusetzen. Bei normalen Individuen liegt die Konzentration des Antigens im Bereich 5 bis 20 ng/ml und kann sich bei Erkrankungen mit Basalmembranveränderungen (Diabetes, Tumoren) signifikant erhöhen.

Neben der Bestimmung von Antigen in biologischem Material läßt sich das Verfahren auch zum Nachweis von Antikörpern (bevorzugt Autoantikörpern) gegen das Antigen heranziehen. Dazu werden verschiedene Verdünnungen der potentiellen Antikörperquellen, wie z. B. Serum mit dem markierten Antigen wie bereits beschrieben, inkubiert und die Antigen-Antikörper-Komplexe abgetrennt und auf die gebundene Menge an Antigen analysiert. Als Vergleich dazu werden analoge Bindungsversuche mit einem Normalserum durchgeführt (Negativkontrolle). Der Unterschied in der Bindungsaktivität gegenüber dieser Negativkontrolle wird dann als Maß für das Vorliegen von Autoantikörpern bewertet.

Die folgenden Beispiele erläutern die Erfindung weiter.

Beispiel   1

Herstellung der globulären Domäne NC1 aus Humangewebe

1 kg Placenta, Niere oder Lunge werden in 5 l 0,5 M
KCl, 0,1 M Tris-HCl pH 7,4 homogenisiert und 24 Stunden
bei 4°C gerührt. Der verbleibende Rückstand wird dann
je einmal mit 2 l bzw. 1 l 6 M Guanidin-Hydrochlorid
bei 4°C extrahiert. Alle Extraktionspuffer enthalten
p-Hydroxymercuribenzoat und Phenylmethansulfonylfluorid
(je 20 mg/l) als Proteaseinhibitoren. Der verbleibende
Rückstand wird dann gegen Wasser dialysiert und
lyophilisiert. Ca. 20 g des lyophilisierten Rückstandes
werden in 800 ml 0,2 M NaCl, 0,002 M CaCl$_2$, 0,05 M
Tris-HCl pH 7,4 homogenisiert und mit 8 mg bakterieller
Kollagenase 24 Stunden bei Raumtemperatur abgebaut.
Dieser Abbau wird einmal wiederholt und die vereinigten
Überstände werden nach Zugabe von EDTA (0,005 M) mit
3 M NaCl gefällt. Das Präzipitat wird in 2 M Harnstoff,
0,05 M Tris-HCl pH 8,6 gelöst und über eine Säule von
DEAE Zellulose (2,5 x 25 cm), die im gleichen Puffer
äquilibriert ist, gegeben. Das nicht an die Säule gebundene Material wird gegen 0,2 M Ammoniumbikarbonat pH
7,9 dialysiert und nach Zugabe von 5 bis 10 mg Kollagenase nochmals 24 Stunden bei 37°C abgebaut und anschließend
lyophilisiert. Die endgültige Reinigung erfolgt dann in
einer Agarose A1,5 m Säule in 1 M CaCl$_2$, 0,05 M Tris-HCl
pH 7,4. Das so erhaltene Produkt weist die in der
folgenden Tabelle angegebene Aminosäurenzusammensetzung
auf.

T A B E L L E

Aminosäurezusammensetzung der globulären Domäne NC1 von
Basalmembrankollagen isoliert aus menschlicher Placenta,
Niere oder Lunge.

| | Placenta | Niere | Lunge |
|---|---|---|---|
| Hydroxyprolin | 4 | 8 | 5 |
| Asparaginsäure | 66 | 67 | 66 |
| Threonin | 60 | 55 | 56 |
| Serin | 102 | 120 | 123 |
| Glutaminsäure | 91 | 104 | 104 |
| Prolin | 67 | 61 | 64 |
| Glycin | 88 | 113 | 105 |
| Alanin | 69 | 73 | 76 |
| Cystein | 41 | 37 | 36 |
| Valin | 42 | 33 | 35 |
| Methionin | 17 | 22 | 19 |
| Isoleucin | 44 | 40 | 43 |
| Leucin | 64 | 71 | 70 |
| Tyrosin | 32 | 38 | 39 |
| Phenylalanin | 37 | 39 | 39 |
| Histidin | 29 | 31 | 32 |
| Hydroxylysin | 6 | 8 | 8 |
| Lysin | 25 | 26 | 27 |
| Arginin | 51 | 54 | 53 |
| Tryptophan | 65 | n.b. | n.b. |

Beispiel    2

Herstellung der markierten Domäne NC1 (=Antigen)

25 µg der nach Beispiel 1 erhaltenen globulären Domäne
NC1 in 0,05 ml 0,1 M Natriumborat pH 8,5 werden mit 1
mCi Jod 125-markiertem Bolton-Hunter Reagenz (3-Jod-1p-
hydroxyphenylpropionsäure-N-hydroxy-succinimidester) 45
Minuten bei 4°C inkubiert. Die Reaktion wird durch
Zugabe von 0,2 ml 0,2 M Glycin in 0,1 M Boratpuffer pH
8,5 gestoppt. Nicht-gebundenes Reagenz wird dann durch
Dialyse gegen Puffer oder durch Gelfiltration an BioGel
P2 entfernt.

Beispiel    3

Durchführung der immunologischen Bestimmung (Radioimmuntest)

Die Konzentration der globulären Domäne NC1 in einer
unbekannten Serumprobe wird in folgendem Inhibitionstest bestimmt:

Eine bestimmte Menge des durch zweimalige Injektion von
0,15 mg NC1 in komplettem Freund'schen Adjuvans in
Kaninchen gewonnenen spezifischen Antikörpers oder
Antiserums wird mit der unbekannten Probe 16 Stunden
bei 4°C vorinkubiert und nach Zugabe von 1 ng markiertem Antigen gemäß Beispiel 2 weitere 8 Stunden bei 4°C
inkubiert. Danach wird ein Überschuß von Antikörper
gegen Kaninchen-Immunoglobulin G zugesetzt und nach
weiteren 16 Stunden bei 4°C das im Immunkomplex gebundene Antigen durch Zentrifugation abgetrennt. Die Inhibitionsaktivität der unbekannten Probe wird mit der
Aktivität einer Standardkonzentration nicht nicht-markiertem Antigen verglichen.

Patentansprüche

1. Humane globuläre Basalmembrankollagen-Domäne NC1,
gekennzeichnet durch ein Molekulargewicht von etwa
170000 Dalton, bestimmt durch Gleichgewichts-
Ultrazentrifugation und Lichtstreuung, hexamere
Struktur mit monomeren Untereinheiten von Molekulargewicht etwa 28000 Dalton und einem Kohlenhydratgehalt von etwa 2 %.

2. Humane globuläre Basalmembrankollagen Domäne NC1
gemäß Anspruch 1,        d a d u r c h
g e k e n n z e i c h n e t,     daß sie in markierter Form vorliegt.

3. Verfahren zur Gewinnung und Reinigung der globulären Basalmembrankollagen-Domäne NC1,
d a d u r c h        g e k e n n z e i c h n e t,
daß man menschliches oder tierisches Gewebe einer
ersten limitierenden Behandlung mit bakterieller
Kollagenase unterwirft, die erhaltenen Abbauprodukte
durch Chromatographie an schwach basischem Anionenaustauscher von nichtkollagenen Proteinen abtrennt,
die kollagenen Abbauprodukte dann einem zweiten
Kollagenaseabbau bei erhöhter Temperatur unterwirft
und die globuläre Domäne NC1 durch Molekularsiebfraktionierung reinigt.

4. Verfahren nach Anspruch 3,        d a d u r c h
g e k e n n z e i c h n e t,     daß man ein
Gewebe verwendet, welches in salzhaltiger Pufferlösung homogenisiert wurde.

5. Verfahren nach Anspruch 3 oder 4,
   d a d u r c h   g e k e n n z e i c h n e t ,
   daß man ein Gewebe verwendet, welches mit Guanidinhydrochlorid in Anwesenheit eines Proteaseinhibitors
   extrahiert wurde.

6. Verfahren nach einem der Ansprüche 3 bis 5,
   d a d u r c h   g e k e n n z e i c h n e t ,
   daß man humanes Placenta-, Nieren- oder Lungengewebe oder Gewebe von Rinderaorten oder EHS-Sarcomgewebe verwendet.

7. Verfahren nach einem der Ansprüche 3 bis 6,
   d a d u r c h   g e k e n n z e i c h n e t ,
   daß die erste Kollagenasebehandlung bei einer
   Temperatur von 15 bis 25°C durchgeführt wird.

8. Verfahren nach einem der Ansprüche 3 bis 7,
   d a d u r c h   g e k e n n z e i c h n e t ,
   daß die zweite Kollagenasebehandlung bei einer
   Temperatur von 30 bis 45°C durchgeführt wird.

9. Verfahren nach einem der Ansprüche 3 bis 8,
   d a d u r c h   g e k e n n z e i c h n e t ,
   daß als schwach basisches Anionenaustauschermaterial
   ein mit Diethylaminoethanol-Gruppen modifiziertes
   Kohlehydrat verwendet wird.

10. Verwendung von gegen humane globuläre Basalmembrankollagen-Domäne NC1 gemäß Anspruch 2 gerichteten
    Antikörpern zur Bestimmung von Basalmembrankollagen
    in Körperflüssigkeiten nach der Methode des
    Radioimmunassay (RIA) oder des Enzymimmunassays
    (EIA).

0155676

11. Verwendung gemäß Anspruch 10,      d a d u r c h
    g e k e n n z e i c h n e t ,      daß die Anti-NC1-
    Antikörper in Form von Antiserum, von isolierten
    Antikörpern oder von monoklonalen Antikörpern eingesetzt werden.

12. Verwendung gemäß Anspruch 10 oder 11,
    d a d u r c h           g e k e n n z e i c h n e t ,
    daß man zusätzlich gegen Immunoglobulin G der für
    die Gewinnung des Anti-NC1-Antikörpers verwendeten
    Tierart gerichtete Antikörper einsetzt.

13. Verwendung von humaner markierter globulärer
    Basalmembrankollagen-Domäne NC1 nach Anspruch 2
    zum Nachweis von hiergegen gerichteten Antikörpern
    in Körperflüssigkeiten nach der Methode des
    Radioimmunassay (RIA) oder Enzymimmunassay (EIA).